(19) Europäisches Patentamt
European Patent Office
Office européen des brevets

(11) Veröffentlichungsnummer: **0 011 769**
**B1**

(12) # EUROPÄISCHE PATENTSCHRIFT

(45) Veröffentlichungstag der Patentschrift: **14.04.82**

(21) Anmeldenummer: **79104484.5**

(22) Anmeldetag: **14.11.79**

(51) Int. Cl.³: **C 07 D 249/08**, C 07 D 233/60, A 61 K 31/41, A 61 K 31/415 // C07C33/46, C07C49/233

(54) **Hydroxyethyl-azole, Verfahren zu ihrer Herstellung sowie ihre Verwendung als Arzneimittel.**

(30) Priorität: **25.11.78 DE 2851116**

(43) Veröffentlichungstag der Anmeldung: **11.06.80 Patentblatt 80/12**

(45) Bekanntmachung des Hinweises auf die Patenterteilung: **14.04.82 Patentblatt 82/15**

(84) Benannte Vertragsstaaten: **AT BE CH DE FR GB IT LU NL SE**

(56) Entgegenhaltungen: **DE-A-2 623 129**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Regel, Erik, Ing. grad., Bergerheide 72 a, D-5600 Wuppertal (DE)**
Erfinder: **Büchel, Karl Heinz, Prof. Dr., Bergerheide 62, D-5600 Wuppertal 1 (DE)**
Erfinder: **Haller, Ingo, Dr., Viktoriastrasse 99, D-5600 Wuppertal 1 (DE)**
Erfinder: **Plempel, Manfred, Dr., Palkestrasse 5, D-5600 Wuppertal 1 (DE)**

EP 0 011 769 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Hydroxyethyl-azole, Verfahren zu ihrer Herstellung sowie ihre Verwendung als Arzneimittel

Die vorliegende Erfindung betrifft neue Hydroxyethylazole, mehrere Verfahren zu ihrer Herstellung sowie ihre Verwendung als Arzneimittel, insbesondere als Antimykotika.

Es ist bereits bekannt geworden, dass 1-(β-Aryl)-ethyl-imidazol-Derivate, wie insbesondere das 1-[2,4-Dichlor-β-(2,4-dichlorbenzyloxy)-phenethyl]-imidazolnitrat gute antimykotische Wirkung aufweisen (vergleiche DE-B-1 940 388). Jedoch ist deren Wirkung in vivo, wie insbesondere gegen Candida, nicht immer befriedigend.

Es wurden die neuen Hydroxyethyl-azole der allgemeinen Formel

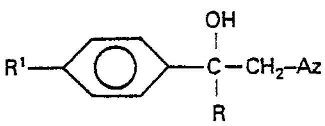 (I)

in welcher

Az für Imidazol-1-yl, 1,2,4-Triazol-1-yl oder 1,3-4-Triazol-1-yl,

R für gegebenenfalls ein- oder zweifach durch Fluor oder Chlor substituiertes Phenyl und

$R^1$ für gegebenenfalls ein- oder zweifach durch Fluor, Chlor, Methoxy oder geradkettiges oder verzweigtes Alkyl mit 1 – 4 Kohlenstoffatomen substituiertes Phenyl steht,

und deren physiologisch verträgliche Säureadditions-Salze gefunden. Sie weisen starke antimykotische Eigenschaften auf.

Weiterhin wurde gefunden, dass man die Hydroxyethyl-azole der Formel (I) erhält, wenn man

a) Azolylmethyl-phenyl-ketone der Formel

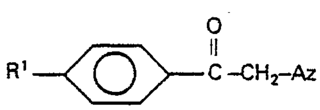 (II)

in welcher

Az und $R^1$ die oben angegebene Bedeutung haben, mit einer Grignard-Verbindung der Formel

R–Mg–X (III)

in welcher

R die oben angegebene Bedeutung hat, und

X für Halogen, insbesondere Chlor oder Brom steht, in Gegenwart eines Verdünnungsmittels umsetzt, oder

b) 1-Halogen-ethan-2-ole der Formel

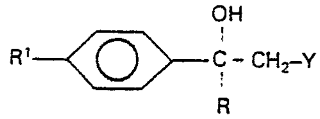 (IV)

in welcher

R und $R^1$ die oben angegebene Bedeutung haben, und

Y für Halogen, insbesondere Chlor oder Brom steht,

mit Azolen der Formel

Z–Az (V)

in welcher

Az die oben angegebene Bedeutung hat, und

Z für Wasserstoff oder ein Alkalimetall steht, gegebenenfalls in Gegenwart eines Säurebindemittels und gegebenenfalls in Gegenwart eines Verdünnungsmittels umsetzt.

Weiterhin können die erfindungsgemäss erhältlichen Hydroxyethylazole der Formel (I) durch Umsetzen mit Säuren in die Salze überführt werden.

Überraschenderweise zeigen die erfindungsgemässen Hydroxy-ethyl-azole neben einer guten antimykotischen in-vitro-Wirksamkeit eine bessere, therapeutisch nutzbare in-vivo-Wirksamkeit gegen Candida als das aus dem Stand der Technik bekannte 1-[2,4-Dichlor-β-(2,4-dichlorbenzyloxy)-phenethyl]-imidazolnitrat, welches ein anerkannt gutes Mittel gleicher Wirkungsrichtung ist. Die erfindungsgemässen Wirkstoffe stellen somit eine wertvolle Bereicherung der Pharmazie dar.

Auch gegenüber der aus DE-A 2 623 129 bekannten Verbindung der Formel

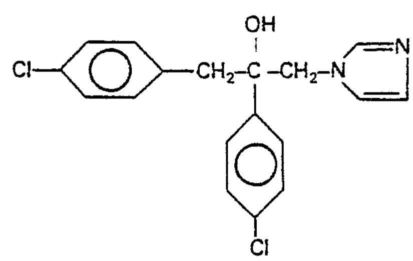

zeigen die erfindungsgemässen Verbindungen bei sonst vergleichbar guter Wirkung bei Trichophyton mentagrophytes und Microsporum canis in vitro eine deutliche Überlegenheit.

Im Einzelnen seien ausser den bei den Herstellungsbeispielen genannten Verbindungen die folgenden Verbindungen der allgemeinen Formel (I) genannt:

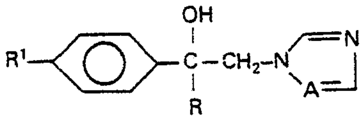

(Ia)

| R | $R^1$ | A |
|---|---|---|
| 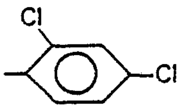 | 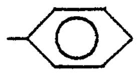 | CH (N) |
| 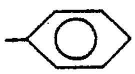 | 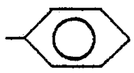 | CH (N) |
| 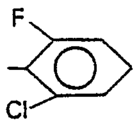 | 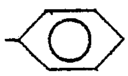 | CH (N) |
| 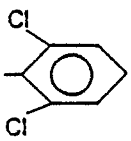 | 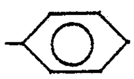 | CH (N) |
| 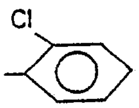 | 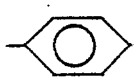 | CH (N) |
| 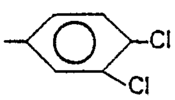 | 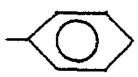 | CH (N) |
| 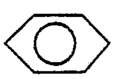 | 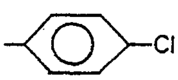 | CH (N) |
| 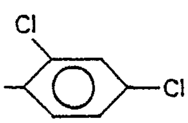 | 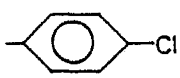 | CH (N) |
| 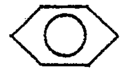 | 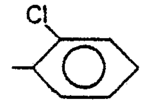 | CH (N) |
| 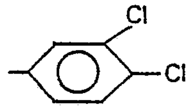 | 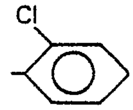 | CH (N) |
| 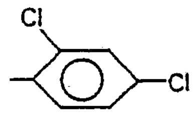 | 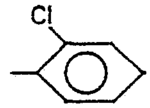 | CH (N) |
| 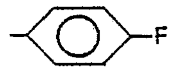 | 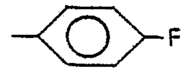 | CH (N) |
| 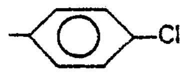 | 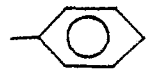 | N |
| 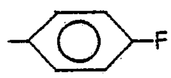 | 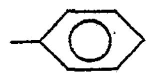 | N |
| 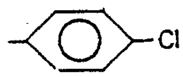 | 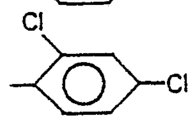 | N |
| 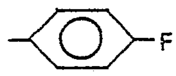 | 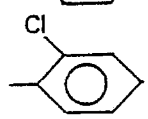 | N |
| 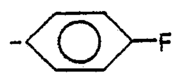 | 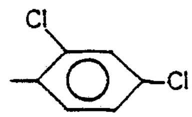 | N |
| 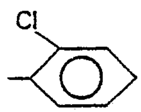 | 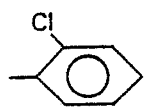 | N |
| 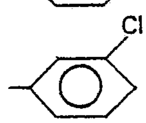 | 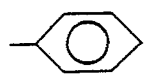 | N |

Verwendet man beispielsweise 4-Biphenylyl-(imidazol-1-yl-methyl)-keton und 4-Chlorphenyl-magnesiumchlorid als Ausgangsstoffe, so kann der Reaktionsablauf durch das folgende Formelschema wiedergegeben werden (Verfahren a):

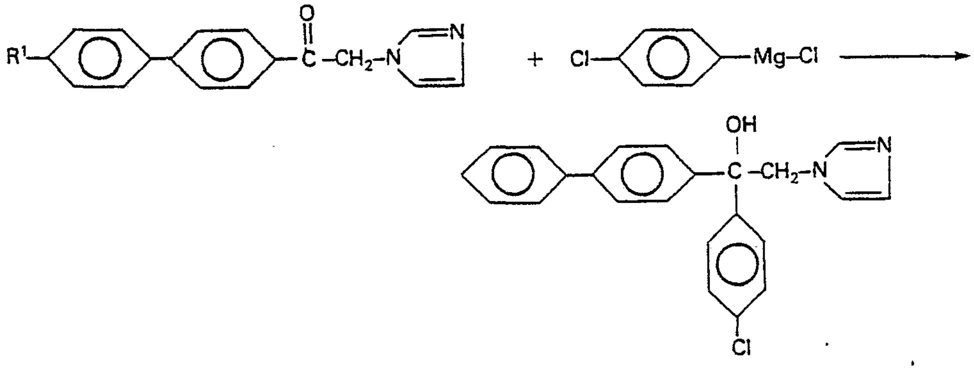

Verwendet man 1-(4-Biphenylyl)-1-(2,4-dichlorphenyl)-2-chlor-ethanol und Imidazol-natrium als Ausgangsstoffe, so kann der Reaktionsablauf durch das folgende Formelschema wiedergegeben werden (Verfahren b):

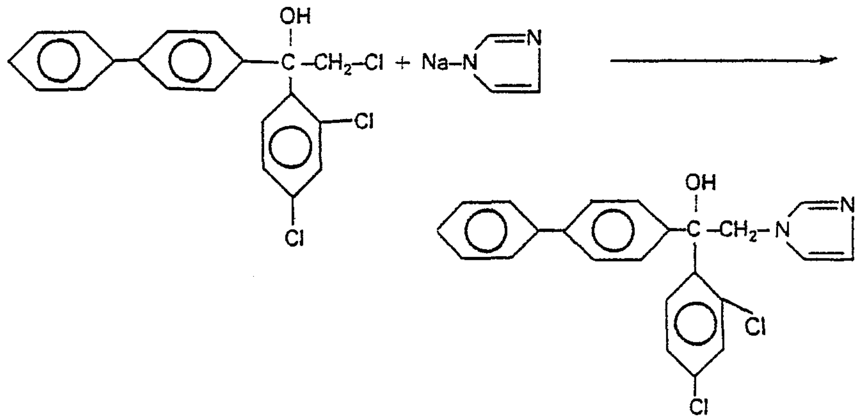

Die für die Verfahrensvariante (a) als Ausgangsstoffe zu verwendenden Azolylmethyl-phenyl-ketone sind durch die Formel (II) allgemein definiert. In dieser Formel stehen Az und $R^1$ für die Reste, die bei den Verbindungen der Formel (I) bereits genannt wurden.

Die Azolylmethyl-phenyl-ketone der Formel (II) sind noch nicht bekannt. Sie lassen sich jedoch in allgemein üblicher und bekannter Weise herstellen, indem man entsprechende Phenacyl-halogenide der Formel

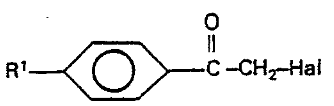

(IV)

in welcher

$R^1$ die oben angegebene Bedeutung hat und Hal für Chlor oder Brom steht,

mit Azolen in Gegenwart eines Verdünnungsmittels, wie beispielsweise Dimethylformamid, und in Gegenwart eines säurebindenden Mittels, wie insbesondere einem Überschuss an Azol, bei Temperaturen zwischen 20 und 80 °C umsetzt (vergleiche hierzu auch die Angaben in der US-Patentschrift 3 658 813).

Als Beispiele für die Ausgangsstoffe der Formel (II) seien genannt:

4-Biphenylyl-imidazol-1-yl-methyl-keton,
4-(4'-Chlorphenylyl)-imidazol-1-yl-methyl-keton,
2-Biphenylyl-imidazol-1-yl-methyl-keton,
4-(2',4'-Dichlorbiphenylyl)-imidazol-1-yl-methyl-keton,
2-Chlor-4-biphenylyl-imidazol-1-yl-methyl-keton,
2-Chlor-4-(4'-Chlorbiphenylyl)-imidazol-1-yl-methyl-keton
sowie die entsprechenden 1,2,4-triazol-1-yl- und 1,3,4-triazol-1-yl-ketone.

Die ausserdem für die Verfahrensvariante (a) als Ausgangsstoffe zu verwendenden Grignard-Verbindungen sind durch die Formel (III) allgemein definiert. In dieser Formel steht R für die Reste, die bei den Verbindungen der Formel (I) bereits genannt wurden.

Die Grignard-Verbindungen der Formel (III) sind allgemein bekannte Verbindungen der organischen Chemie. Als Beispiele seien genannt:
Phenylmagnesiumchlorid, 4-Chlorphenylmagnesiumchlorid, 2,4-Dichlorphenylmagnesiumchlorid, 2,6-Dichlorphenylmagnesiumchlorid, 2-Chlor-6-fluorphenylmagnesiumchlorid, 2-Chlorphenylmagnesiumchlorid, 3-Chlorphenyl-magnesiumchlorid, 3,4-Dichlorphenylmagnesiumchlorid sowie die entsprechenden Bromide.

Die für die Verfahrensvariante (b) als Ausgangsstoffe zu verwendenden 1-Halogen-ethan-2-ole sind durch die Formel (IV) allgemein definiert. In dieser Formel stehen R und $R^1$ für die Reste, die bei den Verbindungen der Formel (I) bereits genannt wurden.

Die 1-Halogen-ethan-2-ole der Formel (IV) sind noch nicht bekannt. Sie lassen sich jedoch in allgemein üblicher und bekannter Weise herstellen, indem man Ketone der Formel (VI) mit Grignard-Verbindungen der Formel (III) entsprechend der Verfahrensvariante (a) umsetzt (vergleiche hierzu auch die Angaben in der DE-OS 2 623 129 sowie die Herstellungsbeispiele).

Die ausserdem für die Verfahrensvariante (b) als Ausgangsstoffe zu verwendenden Azole sind durch die Formel (V) allgemein definiert. In dieser Formel steht Az für die Reste, die bei den Verbindungen der Formel (I) bereits genannt wurden und Z steht vorzugsweise für Wasserstoff, Natrium oder Kalium.

Die Azole der Formel (V) sind allgemein bekannte Verbindungen der organischen Chemie.

Für die erfindungsgemässe Umsetzung nach Verfahren (a) kommen als Verdünnungsmittel alle für eine Grignard-Reaktion üblichen Lösungsmittel in Frage. Hierzu gehören vorzugsweise Ether, wie Diethylether oder Tetrahydrofuran sowie Gemische mit anderen organischen Solventien, wie z.B. Benzol.

Die Reaktionstemperaturen können beim Verfahren (a) in einem grösseren Bereich variiert werden. Im allgemeinen arbeitet man zwischen etwa 2o bis etwa 120 °C, vorzugsweise zwischen etwa 30 bis etwa 80 °C.

Bei der Durchführung des Verfahrens (a) setzt man auf 1 Mol der Verbindung der Formel (II) vorzugsweise einen Überschuss von 3 bis 5 Mol der Grignard-Verbindung der Formel (III) ein. Die Isolierung der Verbindungen der Formel (I) erfolgt in üblicher und bekannter Weise.

Für die erfindungsgemässe Umsetzung nach Verfahren (b) kommen als Verdünnungsmittel vorzugsweise inerte organische Lösungsmittel in Frage. Hierzu gehören vorzugsweise Ketone, wie Diethylketon, insbesondere Aceton und Methylethylketon; Nitrile wie Propionitril, insbesondere Acetonitril; Alkohole, wie Ethanol oder Isopropanol; Ether, wie Tetrahydrofuran oder Dioxan; aromatische Kohlenwasserstoffe, wie Benzol, Toluol und Dichlorbenzol; Formamide, wie insbesondere Dimethylformamid; und halogenierte Kohlenwasserstoffe, wie Methylenchlorid, Tetrachlorkohlenstoff oder Chloroform.

Wird das erfindungsgemässe Verfahren (b) in Gegenwart eines Säurebinders vorgenommen, so kann man alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Di-methylcyclohexylamin, Dicyclohexylmethylamin, N,N-Di-methylbenzylamin, weiterhin Pyridin und Diazobicyclooctan. Vorzugsweise verwendet man einen Überschuss an Azol.

Die Reaktionstemperaturen können beim Verfahren (b) in einem grösseren Bereich variiert werden. Im allgemeinen arbeitet man zwischen etwa 30 bis etwa 200 °C, vorzugsweise bei der Siedetemperatur des Lösungsmittels.

Bei der Durchführung des erfindungsgemässen Verfahrens (b) setzt man auf 1 Mol der Verbindungen der Formel (IV) vorzugsweise 1 bis 2,5 Mol Azol und 1 bis 2,5 Mol Säurebinder ein. Bei Verwendung eines Alkalisalzes setzt man vorzugsweise auf 1 Mol der Verbindung der Formel (IV) 1 bis 1,5 Mol Alkalisalz ein. Zur Isolierung der Verbindungen der Formel (I) wird das Lösungsmittel abdestilliert, der Rückstand direkt oder nach Aufnahme mit einem organischen Solvens mit Wasser gewaschen, die organische Phase gegebenenfalls über Natriumsulfat getrocknet und im Vakuum vom Lösungsmittel befreit. Der Rückstand wird gegebenenfalls durch Destillation, Umkristallisation oder chromatographisch gereinigt.

Zur Herstellung von Säureadditionssalzen der Verbindungen der Formel (I) kommen alle physiologisch verträglichen Säuren in Frage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemässen Verbindungen der Formel (I) und ihre Säureadditions-Salze weisen antimikrobielle, insbesondere antimykotische Wirkungen auf. Sie besitzen ein sehr breites antimykotisches Wirkungsspektrum, insbesondere gegen Dermatophyten und Sprosspilze sowie biphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:
Dermatomykosen und Systemmykosen durch Trichophyton mentagrophytes und andere Trichophytonarten, Mikrosporonarten, Epidermophyton floccosum, Sprosspilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzukker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzugen und Hüllen versehen sein und auch so zusammengesetzt sein, dass sie den oder die Wirkstoffe nur oder bevorzugt in einem bestimmten Teil des Intestinaltraktes, gegebenenfalls verzögert abgeben,

wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Dentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiumetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süssmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemässen Wirkstoffe sowie von pharmazeutischen Zubereitungen, die einen oder mehrere erfindungsgemässe Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 10 bis etwa 300, vorzugsweise 50 bis 200 mg/kg Köpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall. innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Beispiel A
Antimykotische in-vitro-Wirksamkeit

Versuchsbeschreibung:

Die in-vitro-Prüfungen wurden im Reihenverdünnungstest mit Keiminokula von durschnittlich $5 \times 10^4$ Keimen/ml Substrat durchgeführt. Als Nährmedium dienten

a) für Dermatophyten und Schimmelpilze:
Sabouraud's milieu d'épreuve

b) für Hefen:
Fleischextrakt-Traubenzucker-Bouillon.

Die Bebrütungstemperatur betrug 27 °C, die Bebrütungsdauer lag bei 24 bis 96 Stunden.

Bei diesen Tests zeigen die erfindungsgemässen Verbindungen sehr gute minimale Hemmkonzentrationen.

Beispiel B
Antimikrobielle in-vivo-Wirksamkeit (oral) bei Mäuse-Candidose

Versuchsbeschreibung:

Mäuse vom Typ SPF-CF wurden intravenös mit $1 - 2 \times 10^6$ logaritmisch wachsenden Candida-

Zellen, die in physiologischer Kochsalzlösung suspendiert waren, infiziert. Eine Stunde vor und sieben Stunden nach der Infektion werden die Tiere mit jeweils 50 – 100 mg/kg Körpergewicht der Präparate oral behandelt.

Ergebnis:

Unbehandelte Tiere starben 3 bis 6 Tage post infektionem. Die Überlebensrate am 6. Tag post infektionem betrug bei unbehandelten Kontrolltieren etwa 5%.

Die erfindungsgemässen Verbindungen zeigen bei diesem Test Wirkung bis sehr gute Wirkung (60 bis ≥ 90% Überlebende am 6. Tag p.i.), während Miconazol bei diesen Dosierungen keine Wirkung zeigt.

Besonders hervorzuheben ist, dass die erfindungsgemässen Verbindungen teilweise auch bei oraler Therapie der Mäuse-Aspergillose wirksam sind.

Beispiel C

Antimykotische in-vivo-Wirksamkeit (lokal) am Modell der experimentellen Meerschweinchen-Trichophytie

Versuchsbeschreibung:

Weisse Meerschweinchen der Rasse Pirbrightwhite wurden auf dem geschorenen, nicht skarifizierten Rücken mit einer Mikro- und Makrokonidien-Suspension von Trichophyton mentagrophytes infiziert. Bei unbehandelten Tieren entwickelt sich innerhalb 12 Tagen p.i. das typische Bild einer Dermatophytose mit Rötung, Schuppung und Haarausfall bis zum totalen Integument-Defekt an der Infektionsstelle. Die infizierten Tiere wurden – beginnend mit dem 3. Tag p.i. – 1 mal täglich mit 1%igen Polyethylenglykol-Lösungen der erfindungsgemässen Präparate lokal behandelt.

Am 14. Tag p.i. zeigten die unbehandelten Kontrolltiere das typische Bild einer Dermatophytose, während z.B. die Herstellungsbeispiele 1, 2, 4, 8 und 10 den Ablauf der Infektion teilweise bis völlig gehemmt hatten.

Herstellungsbeispiele:

Beispiel 1

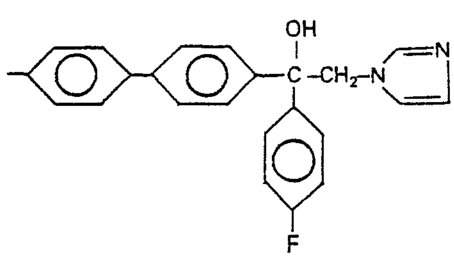

(Verfahren b)

Eine Lösung von 9,5 g (0,175 Mol) Natriummethylat in 49 ml Methylalkohol wird mit 20,2 g (0,297 Mol) Imidazol versetzt. Anschliessend wird eine Lösung von 44,3 g (0,135 Mol) 1-(4-Biphenylyl)-2-chlor-1-(4-fluorphenyl)-ethanol in 103 ml Dimethylformamid zugetropft und 90 Minuten auf 60 °C erhitzt. Die Reaktionsmischung wird durch Abdestillieren der Lösungsmittel im Vakuum eingeengt und der Rückstand mit Wasser verrührt. Die verbleibenden Kristalle werden mit Acetonitril gewaschen und aus Ethylalkohol umkristallisiert. Man erhält 13,5 g (28% der Theorie) 1-(4-Biphenylyl)-1-(4-fluorphenyl)-2-(imidazol-1-yl)-ethanol vom Schmelzpunkt 220 °C.

Herstellung des Ausgangsproduktes

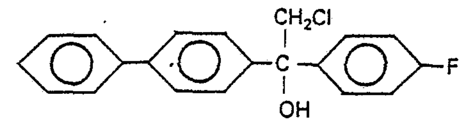

Zu einer Lösung von 4-Fluorphenyl-magnesiumbromid, erhalten aus 7,3 g (0,33 Mol) Magnesium und 52,5 g (0,3 Mol) 4-Fluorbrombenzol in 100 ml Diethylether, werden 34,5 g (0,15 Mol) 4-Phenylphenacylchlorid portionsweise zugegeben. Nach zweistündigem Erhitzen unter Rückfluss wird das Reaktionsgemisch auf wässrige Ammoniumchlorid-Lösung gegossen. Die abgetrennte Etherphase wird mit Wasser gewaschen, über Natriumsulfat getrocknet und eingedampft. Man erhält 44,3 g 1-(4-Biphenylyl)-2-chlor-1-(4-Fluorphenyl)-ethanol.

Beispiel 2

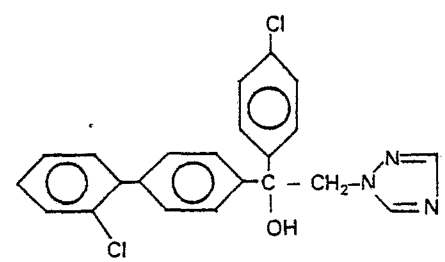

(Verfahren b)

Aus 7,02 g (1,13 Mol) Natriummethylat, 14,96 g (0,22 Mol) Triazol, 40 g (0,1 Mol) 1-(2'-Chlor-4-biphenylyl)-2-chlor-1-(4-chlorphenyl)-ethanol, 36 ml Methylalkohol und 75 ml Dimethylformamid erhält man nach 3-stündigem Erhitzen auf 70 °C analog Beispiel 1 16,2 g (40% der Theorie) 1-(2'-Chlor-4-biphenylyl)-1-(4-chlorphenyl)-2-(1,2,4-triazol-1-yl)-ethanol vom Schmelzpunkt 190 °C.

Herstellung des Ausgangsproduktes

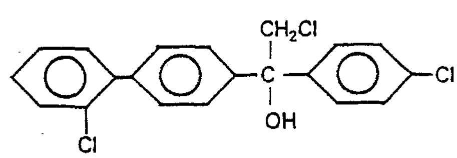

Aus 10,69 g (0,44 Mol) Magnesium, 76,6 g (0,4 Mol) 4-Brom-chlorbenzol und 53 g (0,2 Mol) 4-(2-Chlorphenyl)-phenacyl-chlorid, erhält man analog Beispiel 1 75 g 1-(2'-Chlor-4-biphenylyl)-2-chlor-1-(4-chlorphenyl)-ethanol.

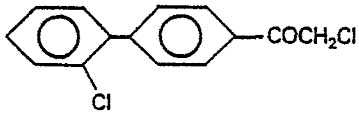

Zu einer Lösung von 377 g (2 Mol) 2-Chlorbiphenyl in 160 ml (2 Mol) Chloracetylchlorid und 1000 ml Methylenchlorid werden 293,7 g (2,2 Mol) Aluminiumchlorid portionsweise eingetragen. Nach 18 Stunden wird das Reaktionsgemisch auf Eis und Salzsäure gegossen. Die organische Phase wird abgetrennt, gewaschen, über Natriumsulfat getrocknet und im Vakuum durch Abdestillieren des Lösungsmittels eingeengt. Das verbleibende Öl wird destillativ gereinigt. Man erhält 478,7 g (90% der Theorie) 4-(2-Chlorphenyl)-phenacylchlorid vom Schmelzpunkt 47 °C.

Beispiel 3

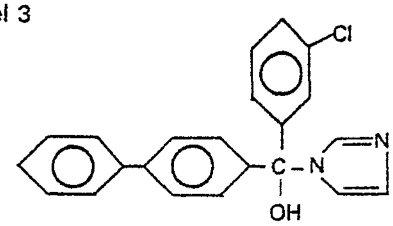

(Verfahren a)

Zu 21,6 g (0,1 Mol) 3-Chlorphenyl-magnesiumbromid in 70 ml Ether (hergestellt aus 2,4 g (0,1 Mol) Magnesium und 19,1 g (0,1 Mol) 3-Bromchlorbenzol) werden portionsweise 13,1 g (0,05 Mol) 4-Biphenylyl-(imidazol-1-yl-methyl)-keton gegeben. Nach Zugabe von 500 ml trockenem Toluol wird der Ether abdestilliert und die entstandene Suspension mit wässriger Ammoniumchloridlösung behandelt. Die Toluolphase wird abgetrennt, filtriert und über Natriumsulfat getrocknet. Man engt durch Abdestillieren des Toluols im Vakuum ein und verrührt den kristallinen Rückstand mit Acetonitril. Nach Umkristallisieren aus Ethanol erhält man 9,4 g (50% d.Th.) 1-(4-Biphenylyl)-1-(3-chlorphenyl)-2-(imidazol-1-yl)-ethanol vom Schmelzpunkt 202 °C.

Tabelle 1

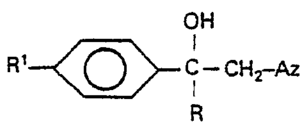

| Bsp. Nr. | R | $R^1$ | Az | Schmelzpunkt (°C) |
|---|---|---|---|---|
| 4 | 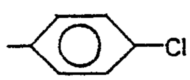 | 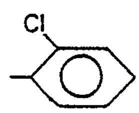 | 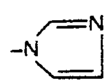 | 187 |
| 5 | 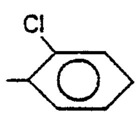 | 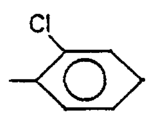 | 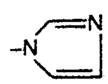 | 206 |
| 6 | 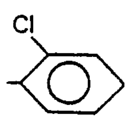 | 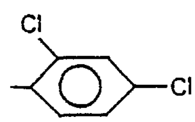 | 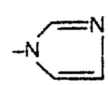 | 220 |
| 7 | 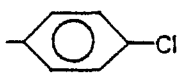 | 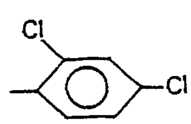 | 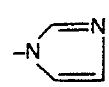 | 176 |
| 8 | 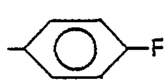 | 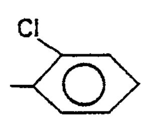 | 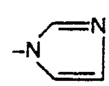 | 100 |
| 9 | 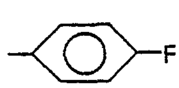 | 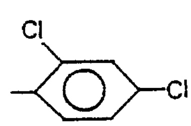 | 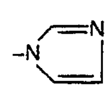 | 225 |
| 10 | 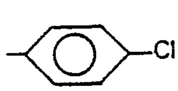 | 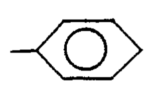 | 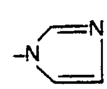 | 230 |

Tabelle 1
(Fortsetzung)

| Bsp. Nr. | R | R¹ | Az | Schmelz-punkt (°C) |
|---|---|---|---|---|
| 11 | 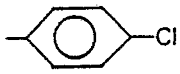 | 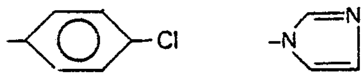 | | 180 |
| 12 | 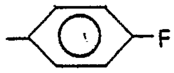 | 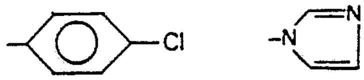 | | 218 |
| 13 | 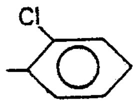 | 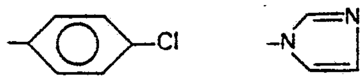 | | 206 |
| 14 | 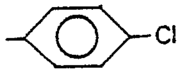 | 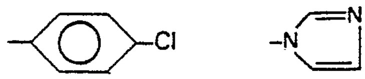 | | 189 |
| 15 | 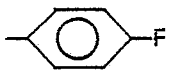 | 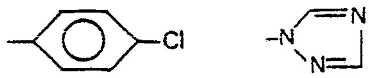 | | 217 |
| 16 | 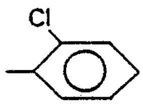 | 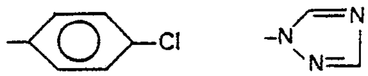 | | 144 |
| 17 | 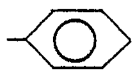 | 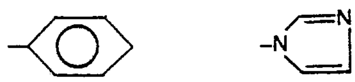 | | 224 |
| 18 | 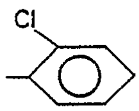 | 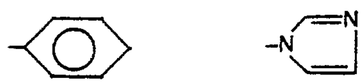 | | 222 |
| 19 | 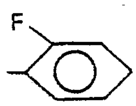 | 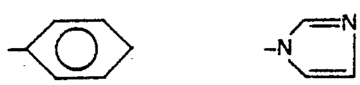 | | 202 |
| 20 | 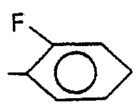 | 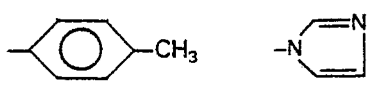 | | 200 |
| 21 | 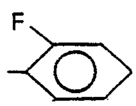 | 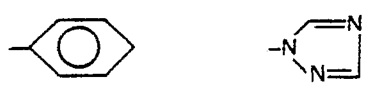 | | 164 |
| 22 | 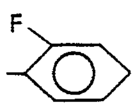 | 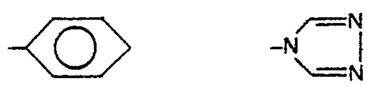 | | 170 |
| 23 | 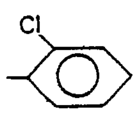 | 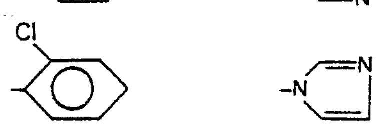 | | 206 |
| 24 | 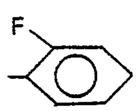 | 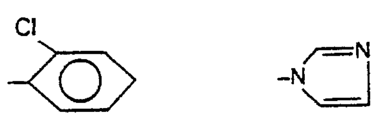 | | 227 |
| 25 | 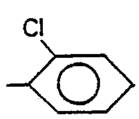 | 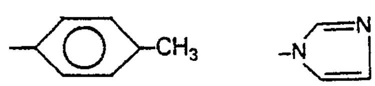 | | |
| 26 | 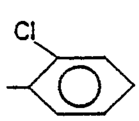 | 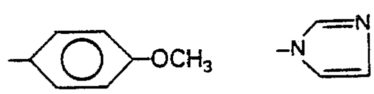 | | |
| 27 | 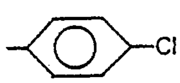 | 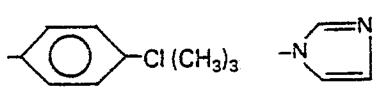 | | |

**Patentansprüche**

1. Hydroxyethyl-azole der Formel

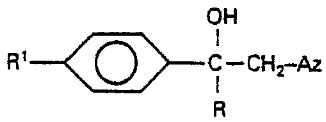

in welcher

Az für Imidazol-1-yl, 1,2,4-Triazol-1-yl- oder 1,3,4-Triazol-1-yl,

R für gegebenenfalls ein- oder zweifach durch Fluor oder Chlor substituiertes Phenyl, und

$R^1$ für gegebenenfalls ein- oder zweifach durch Fluor, Chlor, Methoxy oder geradkettiges oder verzweigtes Alkyl mit 1–4 Kohlenstoffatomen substituiertes Phenyl steht,

und deren physiologisch verträgliche Säureadditions-Salze.

2. Verfahren zur Herstellung von Hydroxyethylazolen der Formel (I), gemäss Anspruch 1, dadurch gekennzeichnet, dass man

a) Azolylmethyl-phenyl-ketone der Formel

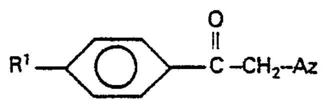

in welcher

Az und $R^1$ die oben angegebene Bedeutung haben, mit einer Grignard-Verbindung der Formel

$R-Mg-X$ (III)

in welcher

R die oben angegebene Bedeutung hat, und

X für Halogen, insbesondere Chlor oder Brom steht, in Gegenwart eines Verdünnungsmittels umsetzt, oder

b) 1-Halogen-ethan-2-ole der Formel

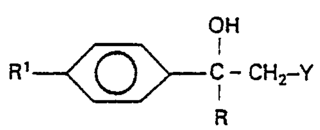
(IV)

in welcher

R und $R^1$ die oben angegebene Bedeutung haben, und

Y für Halogen, insbesondere Chlor oder Brom steht,

mit Azolen der Formel

$Z-Az$ (V)

in welcher

Az die oben angegebene Bedeutung hat, und

Z für Wasserstoff oder ein Alkalimetall steht, gegebenenfalls in Gegenwart eines Säurebindemittels und gegebenenfalls in Gegenwart eines Verdünnungsmittels umsetzt und gegebenenfalls die Verbindungen der Formel (I) durch Umsetzen mit Säuren in die Salze überführt.

3. Arzneimittel, gekennzeichnet durch einen Gehalt an mindestens einem Hydroxyethyl-azol gemäss Anspruch 1.

4. Verfahren zur Herstellung von antimykotischen Mitteln, dadurch gekennzeichnet, dass man Hydroxyethyl-azole gemäss Anspruch 1 mit inerten, nichttoxischen, pharmazeutisch geeigneten Trägerstoffen vermischt.

**Revendications**

1. Hydroxyéthyl-azoles de formule:

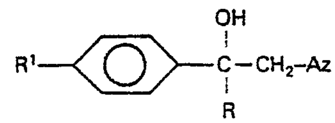

dans laquelle:

Az est le groupe imidazol-1-yle, 1,2,4-triazol-1-yle ou 1,3,4-triazol-1-yle,

R est un reste phényle éventuellement substitué une ou deux fois par du fluor ou du chlore, et

$R^1$ est un reste phényle éventuellement substitué une ou deux fois par du fluor, du chlore, un groupe méthoxy ou un groupe alkyle à chaîne droite ou ramifié ayant 1 à 4 atomes de carbone, et leurs sels d'addition d'acides acceptables du point de vue physiologique.

2. Procédé de production d'hydroxyéthyl-azoles de formule (I), suivant la revendication 1, caractérisé en ce qu'on fait réagir

a) des azolylméthylphénylcétones de formule:

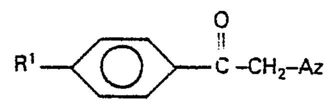

dans laquelle:

Az et $R^1$ ont la définition indiquée ci-dessus, avec un composé de Grignard de formule:

$R-Mg-X$ (III)

dans laquelle:

R a la définition indiquée ci-dessus, et

X est un halogène, en particulier le chlore ou le brome,

en présence d'un diluant,

ou

b) des 1-halogénéthan-2-ols de formule:

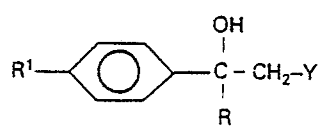
(IV)

dans laquelle:

R et $R^1$ ont la définition indiquée ci-dessus, et

Y est un halogène, en particulier le chlore ou le brome,

avec des azoles de formule:

$Z-Az$ (V)

dans laquelle:

Az a la définition indiquée ci-dessus, et

Z est l'hydrogène ou un métal alcalin, éventuellement en présence d'un accepteur d'acide et, le cas échéant, en présence d'un diluant, et on transforme éventuellement les composés de formule (I) en les sels par réaction avec des acides.

3. Médicament, caractérisé par une teneur en au moins un hydroxyéthyl-azole suivant la revendication 1.

4. Procédé de préparation de compositions antimycosiques, caractérisé en ce qu'on mélange des hydroxyéthyl-azoles suivant la revendication 1 avec des supports inertes non toxiques, acceptables du point de vue pharmaceutique.

**Claims**

1. Hydroxyethylazoles corresponding to the following formula

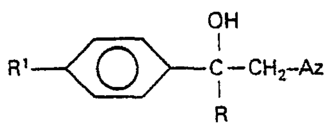

in which

Az represents imidazol-1-yl, 1,2,4-triazol-1-yl or 1,3,4-triazol-1-yl,

R represents phenyl optionally substituted once or twice by fluorine or chlorine and

$R^1$ represents phenyl optionally substituted once or twice by fluorine, chlorine, methoxy or linear or branched $C_1$–$C_4$ alkyl,

and their physiologically compatible acid addition salts.

2. A process for the production of hydroxyethylazoles corresponding to formula (I) in Claim 1, characterised in that

a) azolylmethyl phenyl ketones corresponding to the following formula

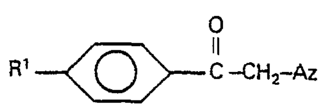

in which

Az and $R^1$ are as defined above,

are reacted with a Grignard compound corresponding to the following formula

$$R-Mg-X \qquad (III)$$

in which

R is as defined above and

X represents halogen, particularly chlorine or bromine, in the presence of a diluent,

or

b) 1-halogen ethan-2-ols corresponding to the following formula

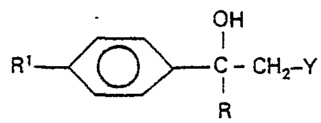

(IV)

in which

R and $R^1$ are as defined above and

Y represents halogen, particularly chlorine or bromine, are reacted with azoles corresponding to the following formula

$$Z-Az \qquad (V)$$

in which

Az is as defined above and

Z represents hydrogen or an alkali methal, optionally in the presence of an acid-binding agent and optionally in the presence of a diluent and the compounds of formula (I) are optionally converted into the salts by reaction with acids.

3. Medicaments containing at least one hydroxyethyl-azole of the type claimed in Claim 1.

4. A process for the production of antimycotic agents, characterised in that hydroxyethyl-azoles of the type claimed in Claim 1 are mixed with inert, non-toxic, pharmaceutically acceptable exipients.